# EUROPÄISCHE PATENTANMELDUNG

(11) **EP 0 887 354 A2**
(43) Veröffentlichungstag der Anmeldung: **30.12.1998**
(21) Anmeldenummer: 98111327.7
(22) Anmeldetag: 19.06.1998
(51) Int. Cl.: C07K 5/068

(54) **Verfahren zur Aufarbeitung von 1- N2-((S)-Ethoxycarbonyl)-3-phenylpropyl)-N6-trifluor-acetyl -L-lysyl-L-prolin (Lisinopril(Tfa)ethylester, LPE)**

(30) Priorität: 23.06.1997 DE 19726520
(71) Anmelder: Degussa Aktiengesellschaft, 60311 Frankfurt (DE)
(72) Erfinder: Tanner, Herbert, Dr., 63457 Hanau (DE); Drauz, Karlheinz, Prof. Dr., 63579 Freigericht (DE); Stingl, Klaus, Dr., 63755 Alzenau (DE); Sator, Gerhard, Dr., 64807 Dieburg (DE); Bethge, Horst, 63517 Rodenbach (DE); Möller, Roland, 63546 Hammersbach (DE); Tacke, Thomas, Dr., 61381 Friedrichsdorf (DE); Rehren, Claus, Dr., 63739 Aschaffenburg (DE)

(57) **Zusammenfassung**

Nach bekannten Verfahren kann LPE der Formel I 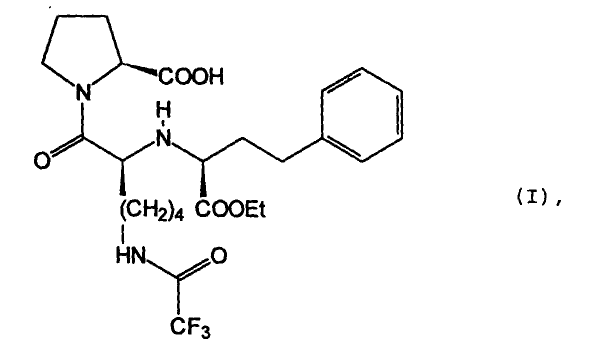 aufgrund seiner schlechten Kristallisierbarkeit nur mit großen Ausbeuteverlusten verbunden hergestellt werden. Zudem sind die Kristalle von I immer mit einem hohen Restlösemittelgehalt behaftet, was ein aufwendiges Trocknungsprozedere erfordert.

Dadurch, daß erfindungsgemäß das Roh-LPE mittels flüssigem oder überkritischem CO₂ getrocknet wird, ist es möglich geworden, die Trocknungszeiten, die vormals im Bereich von ca. 124 h lagen auf ca. 10 h bei gleicher Menge Produkt zu beschränken und dabei ein trockenes LPE zu generieren, welches weniger Restlösemittel enthält und einen geringeren Nebenproduktanteil aufweist als konventionell getrocknetes.

Restlösemittelarmes LPE mit geringem Nebenproduktspektrum.

## Beschreibung

Die Erfindung betrifft ein Verfahren zur Aufarbeitung von 1-[N2-((S)-Ethoxycarbonyl)-3-phenylpropyl)-N6-trifluoracetyl]-L-lysyl-L-prolin (LPE, Verbindung I).

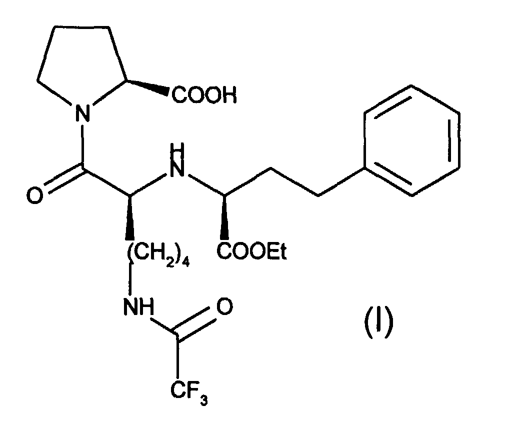

N-substituierte Aminosäuren dieses Typs sind wertvolle Zwischenprodukte für die Herstellung von Inhibitoren des Angiotensin Converting Enzymes (ACE), die als Blutdruckregulatoren wirken. (I) ist das direkte Vorprodukt für 1-[N2-((S)-Carboxy)-3-phenylpropyl)]-L-lysyl-L-prolin (Lisinopril II), welches hervorragende Therapieresultate bei der Bluthochdruckbekämpfung aufweist (Zestril®, Coric®, Prinivil®).

Verbindung (I) erhält man nach dem Stand der Technik durch reduktive Aminierung von 2-Oxo-4-phenyl-buttersäureethylester mit dem Dipeptid Lys(Tfa)-Pro.

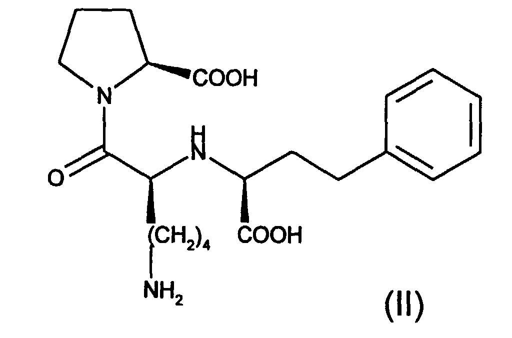

Im J. Org. Chem. 1988, 53,836 - 844 wird ein solches Verfahren beschrieben. (I) wird danach in einer Ausbeute von 42 % durch basische Extraktion der Reaktionsrohlösung, eine nachgeschaltete Extraktion des Produktes in ein organisches Lösungsmittel bei pH 4,6 und anschließende Kristallisation aus Methyl-tert.-butylether, Cyclohexan erhalten.

Die EP 05 23 449 betrifft die Synthese von (I), das gemäß Beispiel 3 mit einer Ausbeute von 60 % gewonnen wird. Die Aufarbeitung der nach diesem Verfahren erhaltenen Reaktionsrohlösung beinhaltet neben dem basischen und einem sauren Extraktionsschritt eine Kristallisation aus Methyl-tert.-butylether. Grundsätzlich sind auch weitere, nicht auf reduktiver Aminierung beruhende, jedoch weniger vorteilhafte Verfahren zur Herstellung von Verbindung (I) bekannt (EP 0 336 368 A2).

Die basische Extraktion - eine Extraktion der wässrigen Produktphase ist notwendig, um Verunreinigungen aus der Aminierungsreaktion zu entfernen - ist jedoch stets mit Produktverlusten durch Amid- und/oder Esterspaltung von (I) nach (II) bzw. von (I) zu Verbindungen (III) und (IV) verbunden.

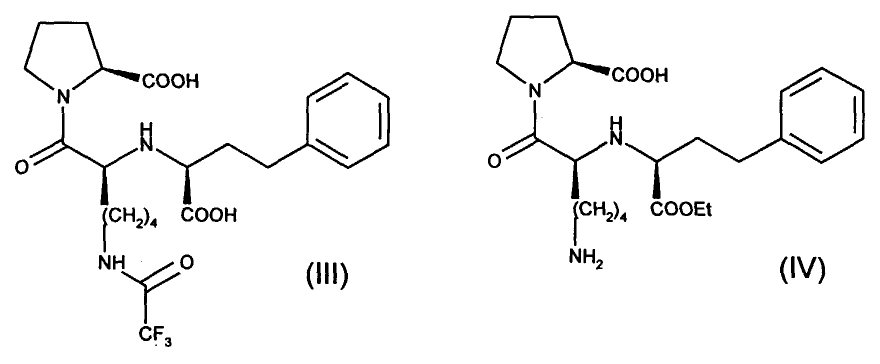

Um diese Produktverluste zu minimieren, muß bei mit tiefen Temperaturen exakt eingestellten pH-Werten und möglichst kurzen Kontaktzeiten gearbeitet werden. Dies ist aufwendig und insbesondere im technischen Maßstab mit Schwierigkeiten verbunden.

Die Kristallisation aus reinem Methyl-tert.-butylether führt zu einem schlecht filtrierbaren Kristallkorn und zu oftmals ungenugenden Ausbeuten (EP 0 645 398 A1). Läßt man (I) aus Lösungen mit hoher Konzentration kristallisieren, wird eine zusätzliche Umkristallisation notwendig. Beschrieben ist auch der Zusatz von Cyclohexan (J. Org. Chem., 1988, 53, 836 - 844) bei der Kristallisation zur Ausbeuteerhöhung. Dabei besteht jedoch die Gefahr der Abscheidung als Öl, was eine Isolierung des Produkts nicht nur im technischen Maßstab stark erschwert.

In EP 0 645 398 A1 wird eingehend die Möglichkeit der Kristallisation von Verbindung (I) aus verschiedenen Lösungsmitteln oder Lösungsmittelgemischen geprüft. Dabei stellte sich heraus, daß beim Einsatz von Methyl-tert.-butylether oder Methyl-tert.-butyletherhaltigen Gemischen der Restlösungsmittelgehalt der Kristalle nach der Kristallisation sehr groß war, bzw. Restlösungsmittel im Kristall eingebunden ist. Dieses erhaltene Rohmaterial an LPE ist extrem schwer zu trocknen. Lange Trocknungszeiten, die die Produktqualität beeinträchtigen können (DKP-Bildung speziell bei erhöhten Temperaturen), bzw. die Neigung zu Anbackungen, machen spezielle aufwendige Trocknungskonzepte notwendig.

Aus WO 95/07928 ist eine Aufarbeitungsart bekannt, die eine Extraktion mit anschließender Kristallisation beschreibt. Hierbei wird in einem pH-Bereich von 0 - 6,3 das Rohmaterial der LPE-Herstellung ggf. mittels mehrerer flüssig/flüssig-Extraktionsschritte vorgereinigt, bevor es aus einem Gemisch von Methyl-tert.-butylether und Methylcyclohexan bei erniedrigten Temperaturen kristallisiert wird.

Durch dieses Vorgehen erhält man ebenfalls, wie oben bereits beschrieben, ein Produkt mit einem sehr hohen Anteil an Restlösungsmittel. Die Schwierigkeiten bei der Isolierung und Trocknung, wie sie oben dargelegt wurden, treffen ebenfalls auf das so erhaltene LPE zu.

Nachteilig bei den bekannten Verfahren ist grundsätzlich die schwierig durchführbare Aufarbeitung, die einer wirtschaftlichen Herstellung der Verbindung (I) zuwiderläuft. LPE (I) ist hinsichtlich der DKP-Bildung, Desethylierung und Desacetylierung empfindlich. Es können zu dessen Aufarbeitung - speziell zu dessen Trocknung - deshalb nur sehr milde Bedingungen (Temperatur, pH-Wert, etc.) angewandt werden. Die Kristallisation, wie sie im Stand der Technik angegeben ist, funktioniert nur mit wenigen Lösungsmitteln/Lösungsmittelgemischen überhaupt, und die erhaltenen Kristalle weisen in jedem Fall eine sehr hohe Restfeuchte bzw. Restlösemittel im Kristall gebunden auf, was unter den erforderlichen milden Bedingungen extrem langes Trocknungsprozedere erfordert. Die langen Trocknungszeiten, gepaart mit der hohen Restfeuchte des Rohmaterials, wirken sich auf die Reinheit des LPE-Endproduktes aber hinsichtlich des Nebenproduktspektrums und der Eigenschaften der Probe in Bezug auf Verbackungen und Verklebungen sowie auf den Restlösemittelgehalt negativ aus.

Aufgabe der Erfindung ist somit, ein verbessertes Verfahren zur Aufarbeitung von aus dem LPE-Herstellprozeß erhaltenen Rohmaterial zur Verfügung zu stellen, welches die üblichen langen, das LPE stressende Trocknungszeiten vermindern hilft.

Aufgabe der Erfindung war es weiterhin, mit dem neuen Verfahren ein LPE-Endmaterial zu generieren, das besonders bezüglich des Restlösungsmittelgehaltes, des Nebenproduktspektrums und der Produkteigenschaften in Hinblick auf Verbackungen und Verklebungen rein ist.

Diese und weitere nicht näher genannte Aufgaben werden durch ein Verfahren der eingangs erwähnten Art gelöst, das durch die Merkmale des kennzeichnenden Teils des Anspruchs 1 beschrieben wird.

Vorteilhafte Verfahrensmodifikationen der Erfindung sind Gegenstand der auf Anspruch 1 rückbezogenen abhängigen Unteransprüche.

Dadurch, daß ein Rohmaterial eines LPE-Herstellprozesses, in dem das LPE angereichert vorhanden ist, mit flüssigem oder überkritischem CO₂ behandelt wird, gelingt es äußerst vorteilhaft, die bislang nicht befriedigenden Trocknungszeiten (1 Woche pro Batch) extrem zu verkürzen (10 h pro Batch) und ein qualitativ hochwertiges Endmaterial (I) bereitzustellen. Diese nicht ohne weiteres vorhersehbare Verbesserung des Aufarbeitungsprozesses ist mit ursächlich für die Entwicklung eines wirtschaftlich interessanten Verfahrens zur Herstellung des eingangs erwähnten blutdrucksenkenden Mittels der Formel (II).

Als aufzuarbeitendes Rohmaterial des LPE-Herstellprozesses kann beispielsweise die vom Lösungsmittel teilweise oder überwiegend befreite Reaktionsmischung des finalen Reaktionsschrittes oder eine schon durch konventionelle Extraktion und/oder Kristallisation vorgereinigte LPE-Charge dienen, in denen das LPE (I) zumindest angereichert vorhanden ist. Das Behandeln dieses Rohmaterials mit flüssigem oder überkritischem CO₂ kann vorzugsweise durch eine Extraktion oder Kristallisation mit diesem Lösungsmittel geschehen. Bei der Extraktion bietet sich bei Einsatz eines flüssigen Rohmaterials eine flüssig/flüssig-Extraktion an, welche ggf. kontinuierlich in sogenannten Gegenstromkolonnen durchgeführt werden kann. Dabei werden unerwünschte Bestandteile des Rohmaterials in einer flüssigen Phase gelöst, während das LPE sich in der anderen anreichert. Vorteilhaft ist es darüber hinaus aber auch, schon festes Rohmaterial, welches durch eine konventionelle Kristallisation gewonnen wurde und welches deshalb einen sehr hohen Restlösungsmittelgehalt aufweist, mittels einer fest/flüssig-Extraktion zu reinigen. Die Verunreinigungen werden dann bevorzugt in CO₂ als Lösungsmittel gelöst und mit dem Strom des CO₂ durch das Rohmaterial mitgerissen.

Das feste Material wird allerdings durch eine dazu geeignete Vorrichtung zurückgehalten, wodurch der gewünschte Reinigungseffekt zustandekommt.

Eine andere bevorzugte Aufarbeitungsmethode des Verfahrens ist das Behandeln des Rohmaterials mit flüssigem oder überkritischem CO₂ durch Kristallisation. Hierbei kann als eine vorteilhafte Variante flüssiges Rohmaterial in CO₂ als Lösungsmittel eingetragen werden oder umgekehrt. Dabei kommt es jedoch zu einer Änderung des Löslichkeitsverhaltens des LPEs (I) im Lösungsmittel, das jetzt aus dem ursprünglichen Lösungsmittel des LPE-Herstellprozesses und überkritischem oder flüssigem CO₂ gebildet wird, woraufhin eine Kristallisation eintritt. Genauso bevorzugt ist aber weiterhin, festes evtl. konventionell vorkristallisiertes Rohmaterial mit einem dadurch bedingten hohen Anteil an Restlösungsmittel in flüssigem oder überkritischem CO₂ zu lösen oder zumindest anzulösen und durch Änderung der Lösungseigenschaften des CO₂ für LPE (I) (z. B. durch Druck oder Temperaturänderung) eine Kristallisation einzuleiten.

Dem oben genannten Verfahren ist eigen, daß besagtes Rohmaterial mit flüssigem oder überkritischem CO₂ kontaktiert wird. Das kann batchweise oder kontinuierlich geschehen, wobei das CO₂ als Lösungsmittel das Rohmaterial je nach Fahrweise der Aufarbeitung entweder durchströmt oder umspült.

Beim kontinuierlichen Durchströmen werden unerwünschte Nebenprodukte im CO₂ gelöst und nicht oder schlecht lösliches festes LPE durch geeignete Vorrichtungen zurückgehalten. Während des Umspülens des flüssigen oder festen Rohmaterials mit flüssigem oder überkritischem CO₂ lösen sich ebenfalls nicht gewünschte Verbindungen in dem CO₂, während LPE als gelöste, gereinigte Komponente oder als Feststoff von CO₂ als Lösungsmittel durch geeignete Vorrichtungen separiert wird. Das nach einem der oben beschriebenen Verfahrensvarianten abgetrennte CO₂, welches sich noch in einem flüssigen oder überkritischen Zustand befindet und welches mit den Verunreinigungen aus dem LPE-Herstellprozeß contaminiert ist, kann anschließend in einem abgetrennten Gefaß einer solchen Vorrichtung, wie sie beispielhaft weiter unten beschrieben ist, entspannt werden, so daß die Verunreinigungen sich flüssig oder fest in diesem Gefäß abscheiden. Die Verunreinigungen können so vorteilhafterweise gesammelt und entsorgt oder leicht wiederaufbereitet werden Das jetzt gasförmige CO₂ wird anschließend kondensiert, in einem Vorratsbehälter gesammelt und kann so verflüssigt zur erneuten Aufarbeitung herangezogen werden. So ist es möglich, mit sehr wenig nicht umweltgefärdenden, in einen Kreisprozeß zurückführbaren Lösungsmittel die erfindungsgemäße Aufarbeitung von LPE durchzuführen.

Die oben näher beschriebenen bevorzugten Ausführungsformen zur Aufarbeitung von Rohmaterial des LPE-Herstellprozesses werden erfindungsgemäß in einem Temperaturbereich von 0 - 50 °C, besonders bevorzugt sind 15 - 45 °C und ganz besonders bevorzugt 25 - 33 °C, und dem dadurch vorgegebenen Druckbereich von 50 bar bis 500 bar zur Erlangung von flüssigem oder überkritischem CO₂ durchgeführt.

Die Gasrate, mit der CO₂ im überkritischen oder flüssigen Zustand das eingesetzte Rohmaterial des LPE-Herstellprozesses umströmt oder umspült, liegt zwischen 10 und 40 kg CO₂ pro kg Produkt pro h, besonders bevorzugt sind 5 - 20 kg pro kg Produkt pro h. Die Gesamtmenge an CO₂, welche das Rohmaterial durchströmt oder umspült, ist dabei abhängig von der Art und Menge der Verunreinigung, die entfernt werden soll. Sie liegt üblicherweise bei 3 - 50 kg pro kg eingesetztem Rohmaterial.

Durch Anwendung der oben beschriebenen Aufarbeitungsvarianten bei der LPE-Herstellung ist es gelungen, die Zeiten im technischen Maßstab, die für die konventionelle Aufarbeitung im Bereich von ca. 124 h lagen, auf ca. 10 h bei gleicher Materialmenge zu verkürzen. Diese viel kürzeren Aufarbeitungszeiten in Zusammenhang mit den sehr milden Bedingungen bei der Aufarbeitung, die eine Zersetzung des LPEs vermeiden helfen, führen zu einem qualitativ sehr hochwertigen LPE der Formel I, dessen Restlösemittelgehalt jedoch unter demjenigen eines nach dem Stand der Technik getrockneten LPEs der Formel I bei vergleichbarem Nebenproduktanteil liegt. Ein LPE, das nach dem vorliegenden Verfahren hergestellt wurde, besitzt ein Nebenproduktanteil und Restlösemittelanteil, der besser ist als der eines nach einem Verfahren des Standes der Technik hergestellten LPEs.

Zusammenfassend läßt sich sagen, daß gegenüber dem Stand der Technik ein Verfahren zur Aufarbeitung von LPE-Rohmaterial erfunden wurde, welches aufgrund der kürzeren Verfahrenszeit kostengünstiger, aufgrund des Einsatzes von CO₂ als Lösungsmittel umweltverträglicher ist und darüber hinaus durch seine milden Bedingungen ein reineres LPE zu generieren gestattet.

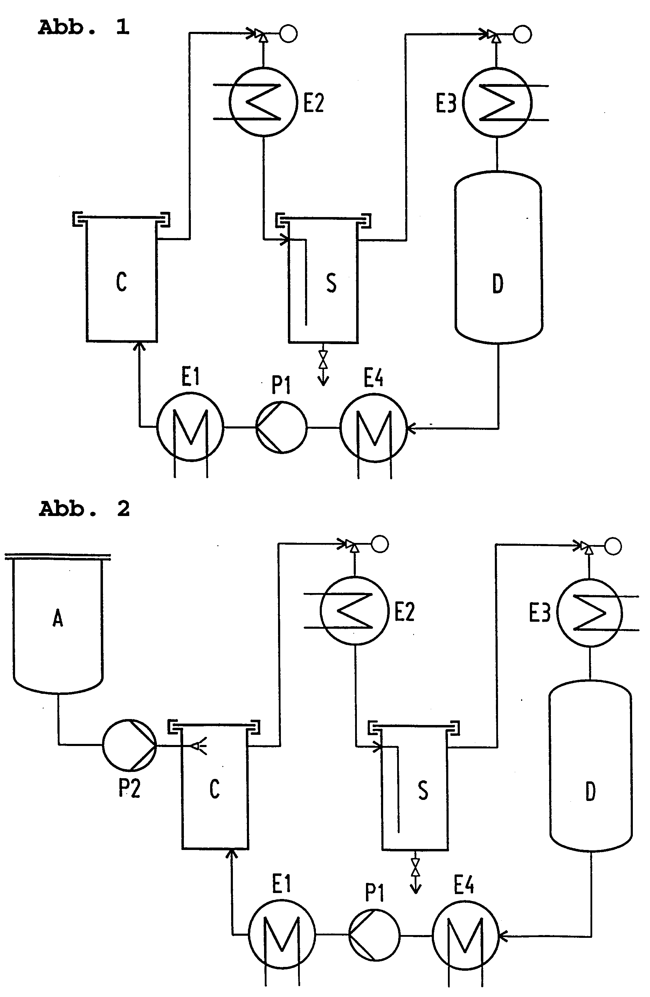

Eine beispielhafte Vorrichtung zur Durchführung des oben beschriebenen Verfahrens ist in Abb. 1 dargestellt.

In dem Gefäß C wird das Rohmaterial vorgelegt. Flüssiges CO₂ aus dem Sammelbehälter D strömt dabei zur Pumpe P, die das CO₂ auf Extraktionsdruck komprimiert und es durch den Wärmeaustauscher E 1, in dem es auf Extraktionstemperatur erwärmt wird, in den Extraktor bzw. in die Extraktionskolonne fördert. Auf dem Weg durch Extraktor bzw. Kolonne C lösen sich die extrahierbaren Stoffe im CO₂. Das mit den gelösten Stoffen beladene CO₂ wird zum Separator S geleitet. Durch Änderung von Druck und/oder Temperatur wird die Lösungsfähigkeit des CO₂ im Separator verringert, so daß die Extrakte dort abgeschieden werden. Die Abscheidung kann in mehreren Stufen erfolgen, so daß man Extraktfraktionen unterschiedlicher Qualitäten erhält. Das gasförmige CO₂ aus dem Separator S wird in einem gekühlten Kondensator verflüssigt und im Sammelbehälter D aufgefangen. Eine weitere Vorrichtung zur Kristallisation gibt die Abb. 2 wieder.

Die Bauteile C, D, E 1 bis 4, P und S entsprechen denen der Abb. 1. Zusätzlich sind noch ein Vorratsbehälter A und eine Pumpe P 2 vorgesehen. In dem Vorratsbehälter A befindet sich eine Lösung des Rohmaterials, die im Unterschied zu der Anlage von Abb. 1 kontinuierlich über eine Pumpe P 2 dem Gefäß C zugeführt wird. Die Führung des CO₂ erfolgt in gleicher Weise wie in Abb. 1, so daß sich ein Gegenstrom zwischen der Lösung des Rohmaterials und dem CO₂ ausbildet.

Die folgenden Beispiele dienen zur Erläuterung der erfindungsgemäßen Aufgabe.

### Aufarbeitung von technisch hergestelltem LPE-Rohmaterial mittels CO₂

Folgende Angaben beziehen sich auf die Versuche 1-3
a) Der Einsatzbehälter wurde bis auf Versuch 3 immer mit 3 kg LPE Roh-Ware befüllt.
b) Der CO₂-Strom durchströmt den Einsatzbehälter von unten nach oben.
c) Nach entsprechenden Durchflußmengen (s. Tabellen 2-4) wurde oberhalb und unterhalb des Einsatzkorbes eine Probe gezogen und per NMR auf den Gehalt an Restlösemitteln vermessen.
d) Der Einsatzbehälter wurde mit einem Mix der in der Tab. 1 genannten LPE-Chargen befüllt.

Zur Durchführung der Trocknungsversuche wurden fünf unterschiedliche, aus einem Produktions-Prozeß stammende zentrifugenfeuchte LPE-Chargen eingesetzt, deren Eingangsanalytik in der Tabelle 1 wiedergegeben ist. Die verwendeten Chargen haben einen Restlösemittelanteil von 24 bis ca. 30 %, mit den Lösungsmittelkomponenten Methyl-tert-butylether [MtBE], Methylcylohexan [MCH].

**Tab.1**

| **Analytik der verwendeten LPE-Chargen** | | | | | |
|---|---|---|---|---|---|
| LPE Charge Rohware | LPE-DKP Gew.[%] | LPE-Desethyl Gew.[%] | dv [(SSS):(RSS)] | Restlosungsmittelgehalte per NMR [%] | |
| | | | | MtBE | MCH |
| **1** | 0.13 | <0.1 | 99.77 : 0.23 | 21.7 | 1.0 |
| **2** | 0.24 | 0.12 | 99.66 : 0.34 | 24.7 | 2.3 |
| **3** | 0.18 | <0.1 | 99.66 : 0.34 | 20.5 | 1.5 |
| **4** | 0.16 | <0.1 | 99.61 : 0.39 | 23.2 | 1.2 |
| **5** | 0.12 | <0.1 | 99.73 : 0.27 | 22.7 | 0.9 |
| **6** | 0.15 | <0.1 | 99.80 : 0.20 | 21.4 | 0.8 |

### Beispiel 1:

Im ersten Versuch (s. Tab. 2) wurde die LPE-Rohware für die Extraktion bei 90 bar in einem Temperaturintervall von 21-26 °C mit CO₂ durchströmt. Nach einer Durchsatzmenge von 15, 30, 45 und 60 kg CO₂wurden entsprechende Proben entnommen und per ¹H-NMR auf Restlösemittelanteile untersucht. Wie aus der Tabelle zu ersehen ist, nimmt die Restlösemittelmenge nach einem CO₂-Durchsatz von 60 kg nahezu gegen Null ab, nur der MtBE-Gehalt wird noch mit >0.1 detektiert. Das Produkt läßt sich nach dem Entspannen in Form einer komprimierten Masse ablassen, daß jedoch an einzelnen Stellen leichte Verklebungen beinhaltet. Das Zerstoßen des abgelassenen Füllguts vollzog sich unproblematisch.

**Tab.2**

| **Extraktion bei 90 bar; Verwendung von 3kg Roh-LPE** | | | | | | | |
|---|---|---|---|---|---|---|---|
| **Vers Nr.** | **Temperatur [°C]** | **Gasrate [kg/h]** | **Druck [bar]** | **Summe CO**_{**2**}**-Menge [kg]** | **Probennahme** | **Restlosemittel [%] per NMR** | |
| 1 | 21-26 | 30 | 90 | 15 | oben | MtBE: | 6.5 |
| | | | | | | MCH: | 0.7 |
| 1 | 21-26 | 30 | 90 | 15 | unten | MtBE: | 5.8 |
| | | | | | | MCH: | 0.2 |
| 1 | 21-26 | 30 | 90 | 30 | oben | MtBE: | n. b. |
| | | | | | | MCH: | n. b. |
| 1 | 21-26 | 30 | 90 | 30 | unten | MtBE: | <0.1 |
| | | | | | | MCH: | <0.1 |
| 1 | 21-26 | 30 | 90 | 45 | oben | MtBE: | 2.2 |
| | | | | | | MCH: | 0.4 |
| 1 | 21-26 | 30 | 90 | 45 | unten | MtBE: | <0.1 |
| | | | | | | MCH: | <0.1 |
| 1 | 21-26 | 30 | 90 | 60 | oben | MtBE: | 0.2 |
| | | | | | | MCH: | 0.1 |
| 1 | 21-26 | 30 | 90 | 60 | unten | MtBE: | <0.1 |
| | | | | | | MCH: | <0.1 |
| n. b.: nicht bestimmt | | | | | | | |

### Beispiel 2:

Im Versuch 2 (s. Tab. 3) wurde nach 30 kg CO₂-Durchsatzmenge der Druck von 90 bar auf 150 bar angehoben. Nach weiteren 30 kg Durchsatz an CO₂ wurde entspannt, und das Produkt konnte in Form eines stangenförmigen Materials aus der Kartusche entnommen werden. Verklebungen am entnommenen Material konnten nur partiell ausgemacht werden. Im Vergleich zum Versuch 1 ist der Gesamtlösemittelanteil ober- und unterhalb der Katusche nahezu identisch.

**Tab.3**

| **Extraktion bei 90-150 bar; 3 kg Roh-LPE** | | | | | | | |
|---|---|---|---|---|---|---|---|
| **Vers Nr.** | **Temperatur [°C]** | **Gasrate [kg/h]** | **Druck [bar]** | **Summe CO**_{**2**}**-Menge [kg]** | **Probennahme** | **Restlösemittel [%] per NMR** | |
| 2 | 25-27 | 30 | 90 | 30 | oben | MtBE: | 6.4 |
| | | | | | | MCH: | 0.3 |
| 2 | 25-27 | 30 | 90 | 30 | unten | MtBE: | 0.15 |
| | | | | | | MCH: | <0.1 |
| 2 | 25-27 | 30 | 150 | 60 | oben | MtBE: | 0.3 |
| | | | | | | MCH: | / |
| 2 | 25-27 | 30 | 150 | 60 | unten | MtBE: | <0.1 |
| | | | | | | MCH: | <0.1 |

### Beispiel 3:

Im Trocknungsversuch Nr. 3 (Tab. 4, 4 kg Einsatzmenge LPE) wurde der Extraktions-druck von 150 bar nach einer Durchstömmenge von 54 kg CO₂ auf 350 bar angehoben. Nach einem CO₂-Durchsatz von insgesamt 80 kg wurde der Druckbehälter entleert.
An diesem LPE-Material konnte hingegen den zuvorigen Versuchen 1-2 keinerlei partielle Verklebungen mehr lokalisiert werden. Auch ließ sich dieses getrocknete LPE leichter zerstoßen als die zuvor generierten Fraktionen und wies keine Kanäle und Verklebungen innerhalb des abgelassenen Materials auf.

**Tab.4**

| **Extraktion bei 150-350 bar; Verwendung von 4 kg Roh-LPE** | | | | | | | |
|---|---|---|---|---|---|---|---|
| **Vers Nr.** | **Temperatur [°C]** | **Gasrate [kg/h]** | **Druck [bar]** | **Summe CO**_{**2**}**-Menge [kg]** | **Probennabme** | **Restlösemittel [%] per NMR** | |
| 4 | 27 | 30 bis 10 kg CO₂, dann 40 | 150 | 54 | oben | MtBE: | 0.6 |
| | | | | | | MCH: | 0.1 |
| 4 | 23-28 | 40 | 150 | 54 | unten | MtBE: | Spur |
| | | | | | | MCH: | / |
| 4 | 23-28 | 40 | 350 | 80 | oben | MtBE: | 0.2 |
| | | | | | | MCH: | Spur |
| 4 | 23-28 | 40 | 350 | 80 | unten | MtBE: | Spur |
| | | | | | | MCH: | / |

### Beispiel 5:

### Vergleichsversuche

Eine zentrifugenfeuchte Roh-LPE Charge wurde folgendermaßen getrocknet.

LPE-Feuchtware (ca. 25-30 %) wird in einem Kontakttrockner innerhalb von 124 h bei Temperaturen von max. 45 °C endgetrocknet.

Anschließend wurde in einer repräsentativen Probe folgender Restlösungsmittelwert festgestellt.

**Tabelle 6**

| | |
|---|---|
| Methylcyclohexan | <0.1 % w/w |
| Methyl-tert.-butylether | 0.5 % w/w |

## Patentansprüche

1. Verfahren zur Aufarbeitung von LPE,
**dadurch gekennzeichnet,**
daß die Aufarbeitung des Rohmaterials eines LPE-Herstellprozesses, in dem LPE angereichert vorhanden ist, durch Behandeln mit flüssigem oder überkritischem CO₂ erfolgt.

2. Verfahren nach Anspruch 1,
**dadurch gekennzeichnet,**
daß man das Rohmaterial extrahiert.

3. Verfahren nach Anspruch 1,
**dadurch gekennzeichnet,**
daß man das Rohmaterial kristallisiert.

4. Verfahren nach Anspruch 2,
**dadurch gekennzeichnet,**
daß man das Rohmaterial mittels flüssig/flüssig-Extraktion, ggf. kontinuierlich in Gegenstromkolonnen, reinigt.

5. Verfahren nach Anspruch 2,
**dadurch gekennzeichnet,**
daß man das Rohmaterial mittels fest/flüssig-Extraktion reinigt.

6. Verfahren nach Anspruch 3,
**dadurch gekennzeichnet,**
daß die Kristallisation des LPEs ausgehend von festem Rohmaterial erfolgt.

7. Verfahren nach Anspruch 3,
**dadurch gekennzeichnet,**
daß die Kristallisation des LPEs ausgehend von gelöstem Rohmaterial erfolgt.

8. Verfahren nach Anspruch 1 bis 7,
**dadurch gekennzeichnet,**
daß flüssiges oder überkritisches CO₂ das Rohmaterial durchströmt und/oder umspült.

9. Verfahren nach Anspruch 8,
**dadurch gekennzeichnet,**
daß das flüssige oder überkritische CO₂ nach dem Durchströmen und/oder Umspülen des Rohmaterials entspannt wird und mitgerissene Verunreinigungen des Rohmaterials in einem Auffangbehälter gesammelt werden.

10. Verfahren nach Anspruch 9,
**dadurch gekennzeichnet,**
daß das entspannte CO₂-Gas anschließend wieder komprimiert und erneut zur Aufarbeitung bereitgestellt wird.

11. Verfahren nach Anspruch 3, 6 und 7,
**dadurch gekennzeichnet,**
daß aus einem Lösungsmittelgemisch kristallisiert wird, welches sich aus Lösungsmitteln des Herstellprozesses und flüssigem oder überkritischem CO₂ zusammensetzt.

12. Verfahren nach einem der vorhergehenden Ansprüche,
**dadurch gekennzeichnet,**
daß CO₂ in einem Druckbereich von 50 bis 500 bar eingesetzt wird.

13. Verfahren nach einem der vorhergehenden Ansprüche,
**dadurch gekennzeichnet,**
daß die Aufarbeitung bei einer Temperatur nicht höher als 45 °C und nicht niedriger als 15 °C erfolgt.

14. Verfahren nach einem der vorhergehenden Ansprüche,
**dadurch gekennzeichnet,**
daß die Rate, mit der CO₂ das Rohmaterial umspült und/oder durchströmt, größer als 5 kg pro kg Produkt pro h und kleiner als 20 kg pro kg Produkt pro h ist.

15. Verfahren nach einem der vorhergehenden Ansprüche,
**dadurch gekennzeichnet,**
daß die Menge, mit der CO₂ das Rohmaterial umspült und/oder durchströmt, größer als 3 kg pro kg Produkt und kleiner als 50 kg pro kg Produkt ist.
